# FASCICULE DE BREVET EUROPEEN

(11) **EP 1 699 873 B1**
(45) Date de publication et mention de la délivrance du brevet: **11.08.2010**
(21) Numéro de dépôt: 04816533.6
(22) Date de dépôt: 10.12.2004
(51) Int. Cl.: C08G 81/02, A61K 8/90, A61Q 3/02, A61Q 5/06

(54) **APPLICATION COSMETIQUE DE COPOLYMERES ROD-COIL**
KOSMETISCHE ANWENDUNG VON ROD-COIL-POLYMEREN
COSMETIC APPLICATION OF ROD-COIL POLYMERS

(30) Priorité: 11.12.2003 FR 0351028; 09.01.2004 US 534999 P
(43) Date de publication de la demande: 13.09.2006
(73) Titulaire: L'Oréal, 75008 Paris (FR)
(72) Inventeur: MOUGIN, Nathalie, F-75011 Paris (FR); SCHULTZE, Xavier, F-77340 Pontault-Combault (FR)
(74) Mandataire: Le Coupanec, Pascale A.M.P.
(86) Numéro de dépôt international: PCT/FR2004/050679
(87) Numéro de publication internationale: WO 2005/059032

(56) Documents cités:
- WO-A-95/22991
- WO-A-03/090727
- LECOMMANDOUX ET AL.: "Self-assembly of Rod-coil diblock oligomers based on alpha-helical peptides" MACROMOLECULES, vol. 34, no. 26, 7 novembre 2001 (2001-11-07), pages 9100-9111, XP002286197
- FLOUDAS, PAPADOPOULOS, KLOK ET AL.: "Hierarchical self assembly of Poly(gamma-benzyl-L-glutamate)-PEG-Poly(ga mma-benzyl-L-glutamate) Rod-coil triblock copolymers" MACROMOLECULES, vol. 36, no. 10, 18 avril 2003 (2003-04-18), pages 3673-3683, XP002286198
- CAILLOL ET AL.: "Synthesis and self-assembly properties of peptide-polylactide block copolymers" MACROMOLECULES, vol. 36, no. 4, 18 janvier 2003 (2003-01-18), pages 1118-1124, XP002286199
- RYUICHIRO ET AL.: "Membrane properties of A_B_A Type block" EUR.POLYM.J., vol. 32, no. 2, 1996, pages 233-237, XP002286200
- LECHNER: "Makromolekulare Chemie" 1993, BIRKHÄUSER , ALLEMAGNE , XP002286201 page 25 - page 37

## Description

La présente invention vise plus particulièrement à proposer l'application de copolymères blocs de type ROD-COIL dans le domaine de la cosmétique.

D'une manière générale, les polymères sont des composés fréquemment mis en oeuvre dans les compositions cosmétiques pour conférer à celles-ci de meilleures propriétés soit en termes de formulation, de tenue dans le temps et/ou de confort pour les utilisateurs.

Ainsi, de nombreuses compositions et en particulier les compositions capillaires dites compositions de « hair styling », qui se présentent sous la forme de nébulisats, de gels ou de mousses, contiennent des résines ou polymères.

Il s'agit en particulier de polymères acryliques ayant des températures de transition vitreuse (Tg) élevées. De tels polymères confèrent notamment aux coiffures un maintien accru. Toutefois, leur trop grande friabilité ne permet pas de garantir une tenue prolongée dans le temps de ce maintien. Une alternative consiste alors à leur associer des composés dits « plastifiants » qui permettent d'abaisser la température de transition vitreuse. Malheureusement, les polymères ont alors tendance à manifester un effet collant et/ou une diminution du coiffant, c'est-à-dire de perdre en partie leurs propriétés mécaniques.

Les polymères constituent également des composants importants dans le domaine des vernis à ongles. Ils contribuent notamment à la formation du film de vernis appliqué à la surface des ongles. Toutefois, comme pour l'application précédente, ce film protecteur, qui contient le polymère, ne s'avère pas toujours suffisamment résistant à l'abrasion mécanique à laquelle sont soumis au quotidien les ongles revêtus du vernis.

En conséquence, aujourd'hui on ne dispose pas d'une solution satisfaisante pour conférer à une composition cosmétique, par le biais de polymères, des propriétés donnant simultanément satisfaction en termes de résistance mécanique et d'effet non collant.

De manière inattendue, les inventeurs ont constaté que des copolymères blocs de type ROD-COIL de composition spécifique permettaient précisément de donner satisfaction à l'ensemble de ces exigences.

Les polymères considérés de la présente invention s'appliquent aisément sur les cheveux et, de façon surprenante, présentent un pouvoir coiffant satisfaisant et s'éliminent facilement par un simple shampooing. Le brossage des cheveux traités ne conduit pas à un effet de poudrage mais laisse les cheveux doux et brillants et non collants.

Les copolymères blocs ROD-COIL sont des copolymères associant d'une part, une ou plusieurs séquences ou bloc de polymères formant un bâtonnet, dit bloc ROD, et caractérisé par une tendance à s'auto assembler en une structure anisotrope et d'autre part, une ou plusieurs séquences ou blocs de polymères sous forme de pelote statistique dit bloc COIL, sujet à de multiples conformations par opposition à un bloc ROD. Ces deux types de blocs ROD et COIL sont liés de façon covalente entre eux.

Ces copolymères blocs ROD-COIL se caractérisent par une miscibilité très importante due à la grande incompatibilité des blocs respectifs ROD et COIL et sont précisément avantageux à ce titre pour obtenir des microstructures et nanostructures à phases séparées importantes.

La demande internationale WO 99/47570 propose ainsi d'utiliser des copolymères blocs ROD-COIL dans un solvant sélectif pour un seul des deux blocs, de manière à solubiliser le bloc concerné et permettre ainsi aux copolymères de type ROD-COIL de s'auto-assembler dans des mésostructures organisées avec une région de blocs insolubilisés et une région de blocs solubilisés.

Ce type de mésostructures est en particulier décrit comme avantageux pour encapsuler des matières actives à l'intérieur de la mésostructure ou pour former des couches d'absorption à la surface d'un substrat.

Les blocs ROD plus particulièrement proposés dans la demande WO 99/47570 sont des polymères incorporant, dans leur structure chimique, plusieurs noyaux aromatiques à l'image de la quinoléine, la pyridine, le thiophène et le benzymidazole par exemple.

Ce sont les interactions Pi, dites encore « Pi-Pi stacking », générées entre ces noyaux aromatiques, qui permettent d'assurer la rigidité des blocs ROD. Plus précisément, l'origine de la faible interaction qui maintient parallèle chaque cycle, est le recouvrement des liaisons Pi entre deux groupes aromatiques de par la délocalisation des électrons. Cette interaction produit un minimum d'énergie stabilisant la structure et conduisant à une orientation parallèle des blocs ROD.

De manière inattendue, les inventeurs ont constaté qu'il était possible de reproduire ces propriétés intéressantes, manifestées par les polymères blocs ROD décrits ci-dessus, en exploitant un autre type d'interaction non covalente.

Plus précisément, la présente invention concerne, selon un de ses aspects, une composition cosmétique contenant dans un milieu physiologiquement acceptable, au moins un copolymère bloc de type ROD-COIL comprenant au moins une structure bloc polymérique à conformation variable dite COIL liée à au moins une structure bloc à conformation limitée dite ROD, **caractérisée en ce que** ladite structure bloc ROD est de nature polymérique et composée en tout ou partie de motif(s) peptidique(s) avec tout ou partie des atomes d'hydrogène libres desdits motifs peptidiques participant à des liaisons hydrogènes non covalentes au sein de ladite structure ROD <page 3-a>.

La présente invention concerne également selon un autre de ses aspects, l'utilisation de copolymères blocs ROD-COIL conformes à l'invention à titre d'agents tensioactifs et/ou d'agents rhéologiques.

Ces copolymères peuvent par ailleurs être utilisés pour leurs propriétés mécaniques, comme polymères filmogènes, en mélange avec d'autres polymères, en tant que renforts de matrices, ou également en mélange avec des molécules en tant que support pour leur encapsulation à l'image par exemple d'agents de coloration et/ou d'agents anti-UV.

### < ladite structure bloc ROD étant ou dérivant de :

- la poly(L-leucine), la poly(L-valine), la poly(phénylalànine),
- la poly(L-lysine) et ses dérivés comme les poly(N-benzyloxycarbonyl-L-lysine) et poly(N-trifluoroacétyl-L-lysine) et leurs sels tels que les chlorhydrates
- le poly(acide L-Glutamique), ses sels, tels que le sel de sodium, et ses dérivés comme les poly γ-alkylesters en C₁-C₃₀ de l'acide L-Glutamique tel que le poly(γ-méthyl-L-Glutamate) ou les poly γ- aryl esters en C₁-C₃₀ ou les poly γ-alkyls aryl esters en C₁-C₃₀ tel que le poly(γ-benzyl-L-Glutamate),
- la polyglutamine et ses dérivés comme les poly(N-hydroxyéthyl-L-glutamine et poly(N-hydroxypropyl-L-glutamine), et
- les copolymères polypeptidiques des monomères précédents du type poly(hydroxyéthyl-L-glutamine/leucine), la poly(hydroxyéthyl-L-glutamine/valine), poly(γ-benzyl-L-Glutamate/leucine), poly(γ-benzyl-L-Glutamate/D,Lphénylalanine), poly(γ-benzyl-L-Glutamate/cinnamylglutamate), poly (N-benzyloxycarbonyl-L-lysine/y-benzyl-L-Glutamate), leurs sels et dérivés ; et le polymère COIL étant choisi parmi :
- les polyéthers de type polyoxyde d'éthylène, polyoxyde de propylène et leurs copolymères,
- les homopolymères de siloxane, tels que par exemple polydiméthylsiloxane (PDMS), et polyméthylphénylsiloxane ou polyméthyllaurylsiloxane,
- les copolymères des différents types de polymères précités,
- les copolymères des différents types de polymères précités avec d'autres copolymères, comme par exemple les polysiloxane/ polyoxyde d'éthylène, et leurs sels et dérivés>

### COPOLYMERE BLOC ROD-COIL

Les copolymères mis en oeuvre dans les compositions selon l'invention conformes à l'invention s'avèrent particulièrement efficaces pour créer des organisations nanoscopiques, en milieu solvant ou non. L'architecture moléculaire ROD-COIL implique en effet une séparation de phase des blocs ROD et des blocs COIL dans des structures à l'échelle submicronique, due à la répulsion de chacun des types de bloc, des contraintes de volume due à la liaison covalente imposée par la connectivité de chacun des blocs et ainsi qu'à l'organisation des blocs ROD.

Ils s'avèrent ainsi capables de s'auto assembler en masse dans une variété de structures supra moléculaires tels que lamellaires, hexagonales, micellaires et vésiculaires, par exemple.

De même, lorsqu'ils sont mis en présence d'un solvant, ils peuvent s'assembler dans une variété de structures supra moléculaires grâce à des interactions entre les segments de polymères et le solvant. Dans ce mode de réalisation, on accède à des structures à l'échelle nanoscopique possédant avantageusement des propriétés mécaniques renforcées pour une faible viscosité, en particulier inférieure à 1 Pa.s (1 000 cps).

La viscosité est mesurée à raison de 20 % en poids de polymère dans l'eau, à 25°C, avec un appareil viscosimètre Brookfield, module aiguille (« spindle »).

La viscosité peut notamment varier de 10⁻³ à 10 Pa.s (1 à 10 000 cps), en particulier de 10⁻³ à 5 Pa.s (1 à 5 000 cps), voire de 5.10⁻³ à 3 Pa.s (5 à 3 000 cps).

La formation d'une morphologie spécifique peut être contrôlée, de façon évidente par la nature des blocs, mais également par la masse globale du polymère, la relative longueur des blocs, la nature du solvant, la concentration en polymère, les additifs et la température.

La nature chimique respective des deux blocs ROD et COIL et en particulier leur incompatibilité favorisent la séparation de phase. La rigidité de l'un des deux blocs peut également favoriser cette séparation de phase.

Conventionnellement, la morphologie adoptée par de tels systèmes dépend du coefficient d'interaction de Flory-Huggins x des différents blocs et donc de leur nature chimique, du degré de polymérisation et de la fraction volumique de chaque bloc. D'une manière générale, plus les chaînes sont longues, plus la séparation de phases est favorisée. Une différence de rigidité de chaînes entre ROD et COIL entraîne donc une augmentation du coefficient d'interaction χ et favorise donc la séparation de phases et la nanostructuration à l'état solide.

Les copolymères blocs ROD-COIL selon l'invention comportent au moins un bloc ROD auto-organisé et au moins un bloc COIL.

Au sens de la présente invention, par « bloc », on entend un enchaînement répétitif d'unités monomères, cette répétition étant au moins égale à 2 unités, notamment à 3 unités, en particulier au moins égale à 5 unités, voire au moins égale à 7 unités.

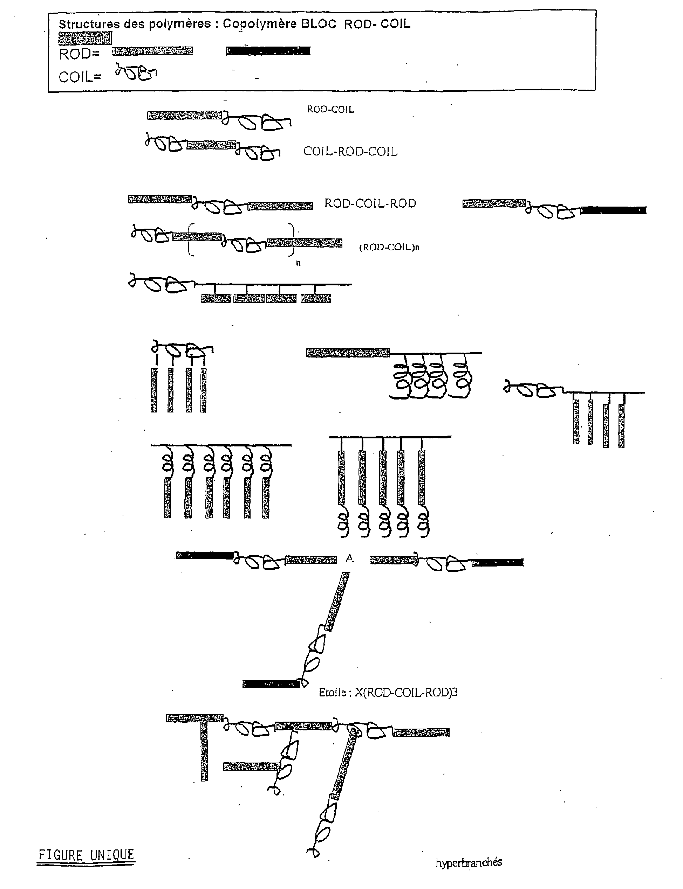

Les copolymères blocs ROD COIL de l'invention peuvent se présenter sous différentes architectures, à savoir di-blocs de type AB avec A qualifiant le bloc ROD et B le bloc COIL, triblocs de type ABA ou BAB, ABC ou multi blocs (AB)ₙ ou (ABC)ₙ avec C désignant un bloc ROD ou COIL de nature différente de A et B. Il peut également s'agir de polymères branchés dont le squelette est constitué de la séquence ROD, ou de polymères branchés dont les greffons sont des ROD, de polymères en étoile. En figure 1, sont schématisés, à titre illustratif et non limitatif de l'invention plusieurs types de structures possibles pour les copolymères conformes à l'invention.

La masse globale en nombre des copolymères ROD-COIL selon l'invention varie d'une manière générale de 700 g/mol à 1 000 000 g/mol, en particulier de 1 000 g/mol à 800 000 g/mol et plus particulièrement de 2 000 g/mol à 500 000 g/mol.

Les copolymères selon l'invention peuvent comporter au moins une séquence réticulable. Selon une variante de l'invention, ils sont avantageusement mis en oeuvre sous une forme non réticulée.

Les copolymères blocs ROD COIL s'avèrent particulièrement avantageux de par leur propriétés mécaniques intéressantes à faible masse et donc de par leur faible viscosité comparativement à des polymères « conventionnels » qui nécessitent de posséder des poids moléculaires élevés pour obtenir les propriétés mécaniques nécessaires.

Qui plus est, ces copolymères blocs ROD-COIL s'avèrent parfaitement compatibles avec une application sous forme de spray. Ils sont donc particulièrement avantageux à ce titre pour une exploitation dans le domaine capillaire.

Enfin, lorsqu'ils possèdent un caractère amphiphile, ils peuvent être aisément véhiculés dans un milieu hydrosoluble ou liposoluble grâce à la séquence qui leur apporte la solubilité désirée.

### BLOC ROD

Les blocs ROD selon l'invention présentent une persistance de trajectoire, c'est-à-dire une anisotropie orientationnelle qui implique un degré de liberté des chaînes limité.

La conformation pseudo rigide, voire rigide des blocs ROD est principalement assurée par des interactions non covalentes imposant une direction à la chaîne et relevant, selon l'invention, de l'existence de liaisons hydrogènes non covalentes manifestées au niveau des atomes d'hydrogène des motifs peptidiques, présents dans les blocs ROD.

Au sens de la présente invention, un motif peptidique est constitué d'au moins une première molécule d'acide aminé liée par une liaison peptidique, encore appelée liaison amide, à une seconde molécule d'acide aminé, identique ou différente. En d'autres termes, ces motifs se caractérisent par la présence dans leur structure d'au moins une liaison peptidique de formule (I) : 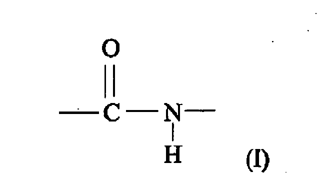 Les interactions de type liaisons hydrogène non covalentes générées par ces liaisons peptidiques, lorsque suffisantes en nombre et/ou placées stratégiquement, permettent de limiter de manière significative le nombre de possibilités d'arrangement dans l'espace des différents monomères, constituant le bloc ROD, les uns par rapport aux autres. Ainsi, la longueur moyenne de la chaîne tenant compte des différentes conformations possibles est plus grande : toutes les restrictions imposées à l'articulation libre des monomères, les uns par rapport aux autres, ont pour effet, en moyenne, d'étirer la chaîne et donc d'augmenter la distance moyenne entre les extrémités de chaîne figurée par <R₀²> _{rod} et pouvant satisfaire à la convention suivante :

<R₀²> _{rod} =CN L²

où
L représente la longueur d'un monomère,
C représente les restrictions imposées à la chaîne avec C supérieur à 1, notamment variant de 4 à 10, et
N représente le nombre de monomères constituant le bloc.

En associant ces blocs ROD à des blocs COIL, on introduit une importante asymétrie conformationnelle dans la même macromolécule. C'est cette conformation asymétrique qui favorise une incompatibilité et améliore donc les forces conductrices pour une séparation nanoscopique et donc un auto-assemblage des copolymères blocs ROD-COIL.

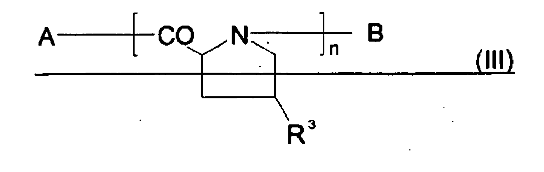

Au sens de la présente invention, sont couverts sous le terme de « dérivés » les sels et leurs dérivés de substitution tels que par exemple les dérivés méthylé, éther, amide et ester.

Sous le terme « sels », on entend les espèces salifiées des amines ou acides. La salification pouvant être réalisées par des sels minéraux ou organiques.

Dans le cas des acides, le neutralisant peut être une base minérale, telle que LiOH, NaOH, KOH, Ca(OH)₂, NH₄OH ou une base organique comme par exemple une alkylamine primaire, secondaire ou tertiaire, telle que la butylamine ou la triéthylamine, Cette alkylamine primaire, secondaire ou tertiaire peut comporter des atomes d'azote et/ou oxygène, et donc comporter par exemple une fonction alcool, par exemple à l'image de l'amino-2-méthyl-2-propanol ou la triéthanolamine.

Dans le cas des amines, conviennent comme neutralisants les sels d'acides minéraux, tels que l'acide sulfurique ou l'acide chlorhydrique, ou des sels d'acides organiques. Ces acides organiques peuvent comporter un ou plusieurs groupes acide carboxylique, sulfonique, ou phosphonique. Ainsi, il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles.

Un exemple d'acide à groupe alkyle est l'acide acétique CH₃COOH.

Un exemple de polyacide est l'acide téréphtalique.

Des exemples d'hydroxyacides sont l'acide citrique et l'acide tartrique.

Le cas échéant, les interactions de type liaisons hydrogènes non covalentes considérées selon l'invention peuvent être remplacées par des interactions d'autres types relevant d'une répulsion stérique, d'une coordination de type acide base de Lewis, d'interactions acide/base, d'interactions dipôles - dipôles, par exemple.

A titre illustratif des systèmes présentant une répulsion stérique, on peut tout particulièrement citer le poly(éthylsiloxane) et le poly(triphénylacrylate).

Les blocs ROD selon l'invention peuvent donc être uniquement constitués de motifs peptidiques tels que définis précédemment ou encore inclure dans leur structure des motifs de nature chimique différente capables d'initier un autre type d'interaction non covalente que les liaisons hydrogène.

Comme précisé précédemment, les polymères bloc ROD conformes à l'invention sont choisis parmi :
- la poly(L-leucine), la poly(L-valine), la poly(phénylalanine),
- la poly(L-lysine) et ses dérivés comme les poly(N-benzyloxycarbonyl-L-lysine) et poly(N-trifluoroacétyl-L-lysine), et leurs sels tels que les chlorhydrates
- le poly(acide L-Glutamique), et ses sels, tels que le sel de sodium, et ses dérivés comme les poly γ-alkylesters en C₁-C₃₀ de l'acide L-Glutamique tel que le poly(γ-méthyl-L-Glutamate) ou les poly γ- aryl esters en C₁-C₃₀ ou les poly γ-alkyls aryl esters en C₁-C₃₀ tel que le poly(γ-benzyl-L-Glutamate),
- la polyglutamine et ses dérivés comme les poly(N-hydroxyéthyl-L-glutamine et poly(N-hydroxypropyl-L-glutamine), et
- les copolymères polypeptidiques des monomères précédents du type poly(hydroxyéthyl-L-glutamine/leucine), la poly(hydroxyéthyl-L-glutamine/valine), poly(γ-benzyl-L-Glutamate/leucine), poly(γ-benzyl-L-Glutamate/D,Lphénylalanine, poly(γ-benzyl-L-Glutamate/cinnamylglutamate), poly (N-benzyloxycarbonyl-L-lysine/γ-benzyl-L-Glutamate), leurs sels et dérivés.

Il est possible de contrôler le caractère hydrophile ou hydrodispersible d'un bloc ROD par la sélection des monomères le constituant.

Ainsi, les blocs ROD de type poly(acide-glutamique) et poly(L-lysine) et leurs sels présentent plus particulièrement un caractère hydrophile.

Selon une variante particulière de l'invention, la masse en nombre des blocs ROD varie de 200 g/mol à 1 000 000 g/mol, en particulier de 250 g/mol à 800 000 g/mol, et plus particulièrement de 250 g/mol à 500 000 g/mol.

Selon une variante particulière de l'invention, les blocs ROD sont présents à raison d'au moins 10 %, en particulier d'au moins 15 %, notamment d'au moins 30 %, et en particulier d'au plus 90 %, notamment d'au plus 85 %, voire d'au plus 80 % en poids par rapport au poids total du copolymère.

Ce rapport peut être en particulier conditionné par la solubilité désirée. Ainsi si le bloc COIL est soluble dans le milieu souhaité alors que le ROD ne l'est pas, la proportion du premier type de bloc COIL peut être avantageusement ajustée à une valeur supérieure.

### BLOC COIL

Par opposition aux blocs ROD décrits précédemment, un bloc COIL est constitué d'un homopolymère ou copolymère dont la chaîne a une conformation géométrique variable aléatoire. Les degrés de liberté des liaisons chimiques entre les monomères constituant la chaîne sont nettement plus nombreux dans le cas d'un bloc COIL que dans le cas d'un bloc ROD, ce qui engendre un nombre de conformations possibles nettement plus élevé.

Par opposition au bloc ROD, la distance moyenne entre les extrémités de chaîne dans le bloc COIL, à savoir <R₀²> _{coil} satisfait à la convention

<R₀²> _{coil} = N L²

avec N et L tels que définis précédemment. 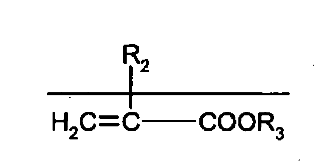 Comme précisé précédemment, les blocs COIL des polymères employés dans les compositions selon l'invention sont composés de :
- polyéthers de type polyoxyde d'éthylène, polyoxyde de propylène et leurs copolymères,
- homopolymères de siloxane, tels que par exemple polydiméthylsiloxane (PDMS), polyméthylphénylsiloxane et polymethyllaurylsiloxane,
- les copolymères des différents types de polymères précités,
- les copolymères des différents types de polymères précités avec d'autres polymères, comme par exemple les polysiloxane/ polyoxyde d'éthylène, et
- leurs sels et leurs dérivés.

Parmi les sels, on peut citer ceux obtenus par neutralisation des groupements acides à l'aide de base minérales telles que LiOH, NaOH, KOH, Ca(OH)₂, NH₄OH ou Zn(OH)₂; ou par une base organique telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol. On peut encore citer la lysine ou la 3-(diméthylamino)-propylamine.

On peut également peut citer les sels d'acides minéraux, tels que l'acide sulfurique, l'acide chlorhydrique, l'acide bromhydrique, l'acide iodhydrique, l'acide phosphorique, l'acide borique. On peut aussi citer les sels d'acides organiques, qui peuvent comporter un ou plusieurs groupes acide carboxylique, sulfonique, ou phosphonique. Il-peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Ces polymères peuvent, le cas échéant, être fonctionnalisés de manière à leur conférer un caractère soluble ou dispersible notamment dans le solvant dans lequel ils sont destinés à être formulés, comme par exemple l'eau et/ou l'éthanol, ou les huiles carbonées, esters, fluorées, silicones.

Au sens de l'invention, un polymère est dit soluble s'il forme une solution liquide, et qualifié de dispersible si à 5 % en poids dans le solvant considéré à 25 °C, il forme une suspension stable de fines particules généralement sphériques.

Conviennent tout particulièrement à l'invention, à titre de bloc COIL, les blocs solubles ou dispersibles dans l'eau et/ou l'éthanol et pouvant comporter des motifs anioniques, cationiques et/ou non ioniques hydrophiles. Les polymères comportant des groupes anioniques ou cationiques peuvent être mis en oeuvre sous une forme neutralisée ou non.

La neutralisation des groupes anioniques peut être effectuée par une base minérale, telle que LiOH, NaOH, KOH, Ca(OH)₂, NH₄OH ou une base organique telle que définie précédemment.

La neutralisation des groupes cationiques peut être effectuée par un acide tel que l'acide sulfurique ou l'acide chlorhydrique, ou un acide organique tel que définis précédemment.

Les groupes cationiques peuvent être quatemisés par des composés à halogène mobile tels que des chlorures ou des bromures d'alkyles en C₁-C₁₂ et notamment le bromure de méthyle ou le chlorure d'éthyle, par le chloroacétate de sodium ou par des sulfones cycliques, par exemple la propanesulfone.

A titre représentatif des polymères comportant des motifs non ioniques, on peut notamment citer les polymères à base d'oxyde d'éthylène, ou de (méth)acrylate de polyoxyde d'éthylène.

Conviennent également à l'invention, des copolymères COIL liposolubles.

A titre représentatif des polymères non hydrosolubles et liposolubles formant le bloc COIL, on peut citer les polydiméthylsiloxanes (PDMS) (polyméthylphénylsiloxane), et/ou de fluorés qui sont solubles dans les silicones ou dérivées.

Dans les copolymères selon l'invention, la masse en nombre du ou des bloc(s) du COIL peut varier de 300 g/mol à 1 000 000 g/mol, en particulier de 500 g/mol à 800 000 g/mol et plus particulièrement de 500 g/mol à 500 000 g/mol.

### SYNTHESE DES COPOLYMERES BLOCS ROD-COIL

Les polymères blocs ROD-COIL selon l'invention peuvent être obtenus par différentes voies de synthèse.

Il peut s'agir d'une réaction entre les deux précurseurs ROD et COIL préformés qui implique la réaction par condensation entre une extrémité fonctionnelle d'un bloc ROD et une extrémité fonctionnelle d'un bloc COIL. Il peut notamment s'agir d'une réaction d'éthérification, de (trans)estérification, de (trans)amidification, d'une formation de liaisons uréthanes, urées, de liaisons C-C, C=C, C=C ou imine.

En ce qui concerne la préparation des blocs ROD conformes à l'invention, la technique de synthèse la plus employée pour obtenir des homo-polypeptides, mais également pour les co-polypeptides, consiste à réaliser l'ouverture de l' α-amino acide N-carboxyanhydride (NCA) correspondant en présence d'un nucléophile, tel qu'une amine primaire ou d'une base forte, comme un alkoxyde de sodium. Cette technique est notamment décrite dans B. Gallot Prog. Polym. Sci., 1035, 21, 1996 et T. J. Deming Macromol. 2970, 35, 2002. Certains métaux de transition peuvent également être utilisés pour réaliser la polymérisation contrôlée de NCA comme décrit dans la demande WO 01/94379.

En ce qui concerne la synthèse des blocs COIL, on pourra notamment se rapporter au document « chimie et physicochimie des polymères » Ed Dunod 2002.

Selon une autre variante, la formation d'un des deux blocs constituant le copolymère bloc ROD-COIL peut être réalisé *in situ* en présence de l'autre bloc déjà constitué. Par exemple, si le bloc COIL est un polymère pouvant être obtenu par voie radicalaire, il suffit de fonctionnaliser l'une des extrémités du bloc ROD par une fonction capable d'amorcer la polymérisation radicalaire contrôlée des monomères considérés pour le bloc COIL. De même, la formation du bloc ROD peut s'effectuer *in situ* en présence d'un bloc COIL et sur une fonction réactive de ce bloc.

Ainsi, lorsque le bloc COIL est un copolymère dérivant de la polymérisation de monomères comprenant des motifs éthyléniques, il peut être obtenu par polymérisation anionique et la fonctionnalisation peut être réalisée par piégeage de l'anion actif par une molécule du type : 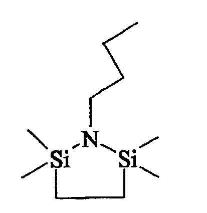 de façon à générer à l'extrémité de la chaîne une fonction amine protégée qui par suite d'une déprotection sera capable d'amorcer la polymérisation du N carboxyanhydride.

Il en est de même si le copolymère dérive de la polymérisation radicalaire. Alors la fonction amine est apportée sur la chaîne via l'amorceur d'une polymérisation radicalaire dite contrôlée. Il peut s'agir par exemple de : H₂N-phenyl-OC(O)C(CH₃)(CH₃)Br. La fonction Br est capable d'amorcer la polymérisation des monomères radicalaires par la méthode dite ATRP.

Selon la fonctionnalité des précurseurs et la répartition des fonctions, il peut être obtenu des diblocs, triblocs, multiblocs, des polymères branchés ou des étoiles.

A titre illustratif des copolymères blocs ROD-COIL selon l'invention, on peut tout particulièrement citer :
- des diblocs ROD-bloc-COIL (encore abrégé ROD-b-COIL) comme par exemple les polyoxyde d'éthylène -b- poly (gamma Benzyl L-glutamate) ; poly(N-benzyloxycarbonyl-L-lysine)-b- polyoxyde d'éthylène ; polyoxyde d'éthylène -b- poly gamma acide L-glutamique, polyoxyde d'éthylène -b- poly (gamma Benzyl L-glutamate), les polydiméthylsiloxane -b-poly(L-acide glutamique) ; polydiméthylsiloxane-b- poly(Benzyl-Glutamate) ; poly(N-benzyloxycarbonyl-L-lysine)-b- poly(oxyde d'éthylène co oxyde de propylène) ; poly(oxyde d'éthylène co oxyde de propylène)-b- poly gamma acide L-glutamique, poly(oxyde d'éthylène co oxyde de propylène)-b- poly (gamma Benzyl L-glutamate) et leurs sels,
- des triblocs COIL-bloc-ROD-bloc-COIL ou ROD-b-COIL-b-ROD comme par exemple les poly(L-acide glutamique)-b-polydiméthylsiloxane-b-poly(L-acidè glutamique) ; poly (gamma Benzyl L-glutamate)-b- poly(oxyde d'éthylène co oxyde de propylène)-b- poly (gamma Benzyl L-glutainate); poly(Benzyl-Glutamate)-b-polydiméthylsiloxane-b-poly(Benzyl-Glutamate) ; poly(L-valine)-b-poly(oxyde d'éthylène co oxyde de propylène)-b-poly(L-valine) et leurs sels.

### APPLICATION DES POLYMERES BLOCS ROD-COIL

Comme précisé précédemment, les polymères blocs ROD-COIL selon l'invention sont particulièrement intéressants pour leur aptitude à conduire à des systèmes tridimensionnels nanostructurés en solution ou en masse. Leurs intéressantes propriétés mécaniques qui se manifestent pour une viscosité réduite sont également particulièrement avantageuses.

Ils peuvent être utilisés pour leur propriété d'adsorption aux interfaces et en particulier pour leurs qualités tensioactives et/ou en tant que véhicule pour l'encapsulation d'actifs.

Ces systèmes peuvent présenter également des qualités rhéologiques intéressantes, et en particulier accroître la viscosité de la formulation les contenant.

Ils peuvent également être mis à profit pour leurs propriétés filmogènes, ou en tant qu'additifs pour les renforts de matrices organiques ou minérales.

Dans les compositions cosmétiques selon l'invention, les copolymères blocs ROD-COIL sont mis en oeuvre à des concentrations compatibles avec un auto-assemblage sous la forme d'une nanostructure. Ces concentrations sont généralement des concentrations supérieures à la concentration micellaire critique ou à la concentration vésiculaire critique. Ces concentrations micellaires critiques ou vésiculaires critiques caractérisent les concentrations au-dessous desquelles le copolymère existe à titre de molécule individuelle ou de chaîne dans la solution et au-dessus de laquelle il y existe à titre d'espèce agrégée. Ces valeurs de concentration critique varient entre 10⁻⁹ mol /l et 10⁻⁴ mol/l.

Conviennent tout particulièrement les concentrations de copolymères variant de 0,5 % en poids à 90 % en poids, en particulier de 0,7 % en poids à 85 % en poids, et plus particulièrement de 0,8 % à 75 % en poids de copolymère(s) par rapport au poids total de la composition.

### MILIEU PHYSIOLOGIQUEMENT ACCEPTABLE

Par « milieu physiologiquement acceptable », on désigne un milieu non toxique et susceptible d'être appliqué sur la peau, les lèvres ou les phanères d'êtres humains. Le milieu physiologiquement acceptable est généralement adapté à la nature du support sur lequel doit être appliquée la composition ainsi qu'à l'aspect sous lequel la composition est destinée à être conditionnée, en l'espèce fluide ou non fluide à la température ambiante et sous pression atmosphérique.

### PHASE AQUEUSE

La composition selon l'invention peut comprendre au moins un milieu aqueux, constituant une phase aqueuse, qui peut former la phase continue de la composition.

La phase aqueuse peut être constituée essentiellement d'eau.

Elle peut également comprendre un mélange d'eau et de solvant organique miscible à l'eau (miscibilité dans l'eau supérieure à 50 % en poids à 25 °C) comme les monoalcools inférieurs ayant de 2 à 5 atomes de carbone tels que l'éthanol, l'isopropanol, le tertio-butanol et le n-butanol, et les alkylèneglycols ayant de 2 à 8 atomes de carbone tels que le propylène glycol 1,3-butylène glycol, le dipropylène glycol, et les cétones en C₃-C₄.

La phase aqueuse (eau et éventuellement le solvant organique miscible à l'eau) peut être présente, à une teneur allant de 1 à 95 % en poids, notamment allant de 3 à 80 % en poids, et en particulier allant de 5 à 60 % en poids par rapport au poids total de la composition.

Un tel milieu peut également comprendre au moins une huile volatile telle que définie ci-après.

### SOLVANTS ORGANIQUES

La composition selon l'invention peut comprendre au moins un milieu solvant organique constituant une phase organique, composé d'au moins un solvant organique volatil à température ambiante. Ces solvants organiques sont plus particulièrement considérés lorsque la composition cosmétique est destinée à une application sur les ongles.

Comme solvant organique volatil ou non à température ambiante, on peut citer :
- les cétones liquides à température ambiante tels que méthyléthylcétone, méthylisobutylcétone, diisobutylcétone, l'isophorone, la cyclohexanone, l'acétone ;
- les alcools liquides à température ambiante tels que l'éthanol, l'isopropanol, le butanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaînes courtes (ayant de 3 à 8 atomes de carbone au total) tels que l'acétate d'éthyle, l'acétate de méthyle, l'acétate de propyle, l'acétate de butyle, l'acétate d'aryle, l'acétate d'isopentyle ;
- les alcanes liquides à température ambiante tels que le décane, l'heptane, l'octane, le dodécane, le cyclohexane, l'isododécane ;
- leurs mélanges.

La composition selon l'invention peut également contenir une ou plusieurs huiles de silicones, généralement en faible quantité, c'est-à-dire pouvant être inférieure à 10 % en poids de la phase solvant. Il peut notamment s'agir d'huiles volatiles ou non telles que diméthicone, phényldiméthicone, alkyldiméthicone, diméthicone, diméthicone copolyol ou cyclométhicone.

Les composés organiques précités peuvent être présents à raison de 0,5 à 99 % en poids par rapport au poids total de la composition.

Lorsque le milieu physiologiquement acceptable comprend une quantité significative en phase organique, celle-ci peut être présente à raison de 30 à 99 % en poids et notamment de 60 à 90 % en poids par rapport au poids total de la composition.

### PHASE GRASSE

La composition, notamment lorsqu'elle est destinée à être appliquée sur les lèvres et la peau, peut comporter au moins une phase grasse et notamment au moins un corps gras liquide à température ambiante (25°C) et à pression atmosphérique et/ou au moins un corps gras solide à température ambiante et à pression atmosphérique tel que les cires, les corps gras pâteux, les gommes et leurs mélanges. La phase grasse peut en outre contenir des agents gélifiants et structurants d'huiles de nature organique et/ou des solvants organiques lipophiles.

La composition peut posséder par exemple une phase grasse continue, pouvant contenir moins de 5% d'eau, notamment moins de 1% d'eau par rapport à son poids total et en particulier peut être sous forme anhydre.

La phase grasse de la composition selon l'invention peut notamment comprendre, à titre de corps gras liquide, au moins une huile volatile ou non volatile ou un de leurs mélanges.

Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).

Par "huile non volatile", on entend une huile restant sur la peau à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées notamment d'origine végétales, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en G₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars^{®} ou de Permetyls^{®}, les esters ramifiés en C₈-C₁₆ tels que le néopentanoate d'iso-hexyle, et leurs mélanges. D'autres huiles hydrocarbonées volatiles comme les distillats de pétrole, notamment ceux vendus sous la dénomination Shell Solt^{®} par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10-6 m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la composition selon l'invention à une teneur allant de 0,1 à 98 % en poids, notamment de 1 à 65 % en poids, et en particulier de 2 à 50 % en poids, par rapport au poids total de la composition.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810^{®}, 812^{®} et 818^{®} par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges,
- les esters de synthèse comme les huiles de formule R¹COOR² dans laquelle R¹ représente le reste d'un acide gras linéaire ou ramifié comportant de 1 à 40 atomes de carbone et R² représente une chaîne hydrocarbonée notamment ramifiée contenant de 1 à 40 atomes de carbone à condition que R¹ + R² soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates.

Les huiles non volatiles peuvent être présentes dans la composition selon l'invention en une teneur allant de 0,01 à 90 % en poids, notamment de 0,1 % à 85% en poids, et en particulier de 1 % à 70 % en poids, par rapport au poids total de la composition.

Plus généralement, le corps gras liquide à température ambiante et à pression atmosphérique peut être présent à raison de 0,01 à 90 % en poids et notamment de 0,1 à 85 % en poids, par rapport au poids de la phase grasse.

En ce qui concerne le corps gras solide à température ambiante et à pression atmosphérique, il peut être choisi parmi les cires, les corps gras pâteux, les gommes et leurs mélanges. Ce corps gras solide peut être présent à raison de 0,01 à 50 %, notamment de 0,1 à 40 % et en particulier de 0,2 à 30 % en poids, par rapport au poids total de la composition.

Ainsi, la composition selon l'invention peut comprendre au moins un composé gras pâteux à température ambiante.

Par "corps gras pâteux" au sens de l'invention, on entend des corps gras ayant un point de fusion allant de 20 à 55 °C, en particulier 25 à 45°C et/ou une viscosité à 40 °C allant de 0,1 à 40 Pa.s (1 à 400 poises), en particulier 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

Plus particulièrement, ces corps gras peuvent être des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées, les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le « THIXINR^{®} » de Rheox.

On peut aussi citer les corps gras pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55°C, comme les stéaryl diméthicones notamment ceux vendus par la société Dow Corning sous les noms commerciaux de DC2503^{®} et DC25514^{®} et leurs mélanges.

Le corps gras pâteux peut être présent dans la composition selon l'invention en une teneur allant de 0,01 à 50% en poids, notamment allant de 0,1 à 45 % en poids, et en particulier allant de 0,2 à 30 % en poids, par rapport au poids total de la composition.

La composition selon l'invention peut comprendre en outre une cire. La cire peut être solide à température ambiante (25°C), à changement d'état solide/liquide réversible, ayant une température de fusion supérieure à 30°C pouvant aller jusqu'à 200°C, une dureté supérieure à 0,5MPa et présentant à l'état solide une organisation cristalline anisotrope. Elle peut être hydrocarbonée, fluorée et/ou siliconée et être d'origine animale, végétale, minérale ou synthétique. Elle peut être choisie par exemple parmi la cire d'abeille, la cire de Carnauba, la cire de Candellila, les cires de paraffine, l'huile de ricin hydrogénée, les cires de silicone, et les cires microcristallines et leurs mélanges.

En particulier, la cire peut être présente sous forme d'émulsion cire(s)-dans-eau.

La cire peut être présente dans la composition selon l'invention en une teneur allant de 0,01 à 50 % en poids, en particulier de 0,1 à 30 % en poids, et notamment de 0,2 à 20 % en poids, par rapport au poids total de la composition.

### AGENTS TENSIOACTIFS

La composition selon l'invention peut en outre contenir des agents tensioactifs émulsionnants présents notamment en une proportion allant de 0,1 à 30 % en poids, et mieux de 5 à 15 % en poids, par rapport au poids total de la composition.

Ces agents tensioactifs peuvent être choisis parmi des agents tensioactifs anioniques, cationiques, amphotères ou non ioniques. On peut se reporter au document «Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis parmi
- les tensioactifs non-ioniques tels que les acides gras et les alcools gras,
- et/ou les tensioactifs anioniques.

### PHASE PARTICULAIRE

La composition de l'invention, peut en outre comprendre une phase particulaire additionnelle pouvant être présente à raison de 0,01 à 40 % en poids, notamment de 0,01 à 30 % en poids et en particulier de 0,05 à 20 % en poids, par rapport au poids total de la composition.

Elle peut notamment comprendre au moins un pigment et/ou au moins une nacre et/ou au moins une charge complémentaires utilisés dans les compositions cosmétiques.

Par pigments, il faut comprendre des particules blanches ou colorées, minérales ou organiques, insolubles dans la phase hydrophile liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées.

Les pigments peuvent être présents dans la composition à raison de 0,01 à 25 % en poids, en particulier de 0,01 à 15 % en poids, et notamment de 0,02 à 5 % en poids par rapport au poids de la composition.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome. Parmi les pigments organiques utilisables dans l'invention, on peut citer le noir de carbone, les pigments de type D & C, et les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537. La quantité et/ou le choix de ces pigments sont généralement ajustés en prenant en compte la quantité en nanotubes présente dans la composition cosmétique considérée.

Les nacres peuvent être présentes dans la composition à raison de 0,01 à 25 % en poids, notamment de 0,01 à 15 % en poids, et en particulier de 0,02 à 5 % en poids, par rapport au poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges complémentaires peuvent être présentes à raison de 0,01 à 40 % en poids, notamment 0,01 à 30 % en poids, et en particulier de 0,02 à 20 % en poids par rapport au poids total de la composition. Leur quantité est également généralement ajustée en prenant en compte la quantité en nanotubes.

Il peut notamment s'agir de charges sphériques comme par exemple le talc, le stéarate de zinc, le mica, le kaolin, les poudres de polyamide (Nylo^{®}) (Orgasol^{®} de chez Atochem), les poudres de polyéthylène, les poudres de polymères de tétrafluoroéthylène (Téflo^{®}), l'amidon, le nitrure de bore, des microsphères polymériques telles que celles de chlorure de polyvinylidène/acrylonitrile comme l'Expancel^{®} (Nobel Industrie), de copolymères d'acide acrylique (Polytrap^{®} de la société Dow Corning), les microbilles de résine de silicone (Tospearls^{®} de Toshiba, par exemple), et les organopolysiloxanes élastomères.

La composition peut comprendre également des colorants hydrosolubles ou liposolubles en une teneur allant de 0,01 à 6 % en poids, par rapport au poids total de la composition, notamment allant de 0,01 à 3 % en poids. Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, et le jaune quinoléine. Les colorants hydrosolubles sont par exemple le jus de betterave et le bleu de méthylène.

La composition selon l'invention peut, de plus, comprendre tous les ingrédients classiquement utilisés dans les domaines concernés et plus spécialement dans le domaine cosmétique et dermatologique. Ces ingrédients sont en particulier choisis parmi les vitamines, les antioxydants, les épaississants, les oligo-éléments, les adoucissants, les séquestrants, les parfums, les agents alcalinisants ou acidifiants, les conservateurs, les filtres UV, les actifs hydrophiles ou lipophiles et leurs mélanges. Les quantités de ces différents ingrédients sont celles classiquement utilisées dans les domaines concernés et par exemple de 0,01 à 20 % du poids total de la composition.

Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction considérée.

La composition de l'invention peut être obtenue selon les procédés de préparation classiquement utilisés en cosmétique ou en dermatologie.

Elle peut se présenter sous forme d'une solution aqueuse ou huileuse, d'une émulsion directe ou inverse.

Elle peut également se présenter sous la forme d'un produit coulé en stick ou en coupelle comme les rouges à lèvres ou les baumes à lèvres, les fonds de teint coulés, les produits anti-cernes, les « correcteurs » et/ou « embellisseurs » de teint et les fards à paupières ou à joues.

Elle peut se présenter sous la forme d'un produit de soin et/ou de maquillage des ongles, de la peau et/ou des lèvres.

Elle peut également se présenter sous la forme d'un produit capillaire pour le soin et/ou le coiffage du cheveu que ce soit pour les laques, sprays, gels, mousses, shampooings, après shampooings.

Il peut s'agir d'une formulation en présence d'agent propulseur qui peut être n'importe quel gaz liquéfiable utilisé habituellement dans des dispositifs aérosol.

La présente invention concerne également un procédé de traitement cosmétique d'au moins une matière kératinique, notamment la peau, les cheveux et/ou les ongles comprenant l'application sur ladite matière d'une composition selon l'invention.

Les exemples de compositions ci-après sont donnés à titre illustratif et sans caractère limitatif.

### EXEMPLE 1

1/ Préparation du polymère bloc polyoxyde de propylène -bloc-poly(gammabenzyl-L-glutamate) :
   - On dissout 2,454g de (Gamma)benzyl-L-glutamate-N-carboxyanhydride dans 60 g de dichlorométhane, sous argon,
   - 0,875g de polypropylène glycol alpha,oméga NH₂ (4000 g/mol) dissous dans 4 g de dichlorométhane sont ajoutés à cette solution à l'aide d'une seringue.
   La réaction a lieu durant 5 jours, à température ambiante.
   Le milieu réactionnel est ensuite précipité dans l'éther éthylique.
   On obtient une poudre blanche qui est séchée sous vide donner 1,86g de polymère, soit un rendement de 62 %.
2/ Préparation du polymère bloc polypropylène glycol-bloc-poly(L-acide glutamique) par hydrolyse du groupement benzyle :
   - on place 1,86 g du polymère synthétisé ci-dessus au reflux dans 15 ml d'acide trifluoroacétique pendant 4h.
   Puis, le polymère est précipité dans l'éther éthylique et séché sous vide.

On récupère 1,2 g de polymère, soit un rendement d'hydrolyse de 70 % mesuré par RMN.

### EXEMPLE 2

### Polyaminoacide-b-poly(oxyde d'éthylène co oxyde de propylène)-b-polyaminoacide

Le poly(oxyde d'éthylène /co oyde de propylène)-α,ω̅ diNH2 (P(OE/PO )-diNH₂) de masse 1000 g.mol-l (1g) est mis en solution dans 10ml de Chloroforme.

Le milieu réactionnel est mis sous balayage d'azote. 0,86g de valine-NCA sont alors ajoutés sur 2h : on observe un dégagement gazeux (CO₂). La réaction est laissée à température ambiante pendant 24h.

Selon ce procédé, sont obtenus les polymères suivants :

| | Prépolymère | NCA^{c} | Rapport Massique ^{d} : P(OE/PO) diNH₂/NCA | Composition du polymère obtenu : rapport massique COIL/ROD^{e} |
|---|---|---|---|---|
| Exemple 2.1 | P(OE/PO) diNH₂^{a} : 2g | valine : 0,86g | 70/30 | 84/16 |
| Exemple 2.2 | P(OB/PO )-diNH₂^{a} : 1.514g | Benzyl-Glutamate : 1.816 | 45/55 | 55/45 |

| | | | | |
|---|---|---|---|---|
| ^{a)} poly(oxyde d'éthylène co oxyde de propylène )-α,ω̅ diNH₂ avec un ratio en moles de PO/EO=3/19 ; de masse 1000 g.mol⁻¹. ^{c)} Dérivé N -CarboxyAnhydride : (voir dessin ci-dessous) ^{d)} Rapport massique dans le mélange de départ. ^{e)} Rapport massique dans le polymère après purification, déterminé par RMN. | | | | |

### EXEMPLE 3

Selon le même procédé que celui décrit précédemment, on obtient les polymères suivants :

| | Prépolymère | NCA^{c} | Rapport Massique^{d} : PDMS diNH₂/ NCA | Composition du polymère obtenu : rapport massique COIL/ROD^{e} |
|---|---|---|---|---|
| Exemple3.1 | PDMSdiNH₂^{b} : 0,28g | Benzyl-Glutamate : 1,35g | 17/83 | 20/80 |

| | | | | |
|---|---|---|---|---|
| ^{b)}Polydiméthylsiloxaneα,ω diNH₂ de masse 10000g.mol⁻¹. ^{c)}N-Carboxyanhydride dont les structures sont données ci-dessous. ^{d)}Rapport massique dans le mélange de départ. ^{e)} Rapport massique dans le polymère après purification, déterminé par RMN. | | | | |

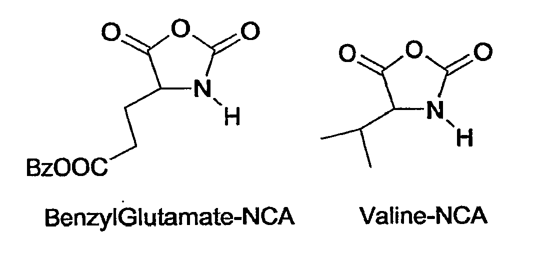

### EXEMPLE 4

### Composition cosmétique capillaire :

Le composé de l'exemple 1 est solubilisé dans de l'eau à raison de 10 % en poids par solubilisation directe.

La solution est mise en flacon pompe et propulsée sur les cheveux. Le spray donne un produit coiffant les cheveux, sans collant.

### EXEMPLE 5

### Composition cosmétique capillaire :

Le composé de l'exemple 2.2 est solubilisé dans de l'eau à raison de 10 % en poids par solubilisation directe.

La solution est mise en flacon pompe et propulsée sur les cheveux. Le spray donne un produit coiffant les cheveux, sans collant

### EXEMPLE 6

### Composition cosmétique pour ongles :

Le composé de l'exemple 3.1 est solubilisé à 20 % en poids dans l'acétate d'éthyle.

Cette solution est appliquée sur les ongles.

Un film brillant est ainsi obtenu non collant.

## Revendications

1. Composition cosmétique contenant, dans un milieu physiologiquement acceptable, au moins un copolymère bloc de type ROD-COIL comprenant au moins une structure bloc polymérique à conformation variable dite COIL liée à au moins une structure bloc à conformation limitée dite ROD, **caractérisée en ce que** ladite structure bloc ROD est de nature polymérique et composée en tout ou partie de motif(s) peptidique(s) avec tout ou partie des atomes d'hydrogène libres desdits motifs peptidiques participant à des liaisons hydrogènes non covalentes au sein de la structure ROD, ladite structure bloc ROD étant ou dérivant de :
- la poly(L-leucine), la poly(L-valine), la poly(phénylalanine),
- la poly(L-lysine) et ses dérivés comme les poly(N-benzyloxycarbonyl-L-lysine) et poly(N-trifluoroacétyl-L-lysine) et leurs sels tels que les chlorhydrates
- le poly(acide L-Glutamique), ses sels, tels que le sel de sodium, et ses dérivés comme les poly γ-alkylesters en C₁-C₃₀ de l'acide L-Glutamique tel que le poly(γ-méthyl-L-Glutamate) ou les poly γ- aryl esters en C₁-C₃₀ ou les poly γ-alkyls aryl esters en C₁-C₃₀ tel que le poly(γ-benzyl-L-Glutamate),
- la polyglutamine et ses dérivés comme les poly(N-hydroxyéthyl-L-glutamine et poly(N-hydroxypropyl-L-glutamine), et
- les copolymères polypeptidiques des monomères précédents du type poly(hydroxyéthyl-L-glutamine/leucine), la poly(hydroxyéthyl-L-glutamine/valine), poly(γ-benzyl-L-Glutamate/leucine), poly(γ-benzyl-L-Glutamate/D,Lphénylalanine, poly(γ-benzyl-L-Glutamate/cinnamylglutamate), poly (N-benzyloxycarbonyl-L-lysine/y-benzyl-L-Glutamate), leurs sels et dérivés ;
et le polymère COIL étant choisi parmi :
- les polyéthers de type polyoxyde d'éthylène, polyoxyde de propylène et leurs copolymères,
- les homopolymères de siloxane, tels que par exemple polydiméthylsiloxane (PDMS), et polyméthylphénylsiloxane ou polyméthyllaurylsiloxane,
- les copolymères des différents types de polymères précités,
- les copolymères des différents types de polymères précités avec d'autres copolymères, comme par exemple les polysiloxane/ polyoxyde d'éthylène, et
- leurs sels et dérivés.

2. Composition selon la revendication précédente, **caractérisée en ce que** la masse en nombre des blocs ROD varie de 200 g/mol à 1 000 000 g/mol, en particulier de 250 g/mol à 800 000 g/mol, et plus particulièrement de 250 g/mol à 500 000 g/mol.

3. Composition selon la revendication 1 ou 2, **caractérisée en ce que** les blocs ROD sont présents à raison d'au moins 10 %, en particulier d'au moins 15 %, notamment d'au moins 30 %, et en particulier à raison d'au plus 90 %, notamment d'au plus 85 %, voire d'au plus 80 % en poids par rapport au poids total du copolymère.

4. Composition selon l'une quelconque des revendications 1 à 3, **caractérisée en ce que** la distance moyenne entre les extrémités de chaîne dans le bloc COIL, à savoir <R₀²> _{coil} satisfait à la convention :
<R₀²>_{coil} N L²,
avec L représentant la longueur d'un monomère et N représentant le nombre de monomères constituant le bloc.

5. Composition selon l'une quelconque des revendications 1 à 4, **caractérisée en ce que** la masse en nombre des blocs COIL varie de 300 g/mol à 1 000 000 g/mol, en particulier de 500 g/mol à 800 000 g/mol, et plus particulièrement de 500 g/mol à 500 000 g/mol.

6. Composition selon l'une quelconque des revendications 1 à 5, **caractérisée en ce que** sa masse globale en nombre varie de 700 g/mol à 1 000 000 g/mol, en particulier de 1 000 g/mol à 800 000 g/mol, et plus particulièrement de 2 000 g/mol à 500 000 g/mol.

7. Composition selon l'une quelconque des revendications 1 à 6, **caractérisée en ce que** ledit copolymère bloc ROD-COIL est non réticulé.

8. Composition selon l'une quelconque des revendications 1 à 7, **caractérisée en que** ledit copolymère ROD-COIL est choisi dans le groupe comportant:
- des diblocs ROD-bloc-COIL (encore abrégé ROD-b-COIL) comme par exemple comme les polyoxyde d'éthylène -b- poly (gamma Benzyl L-glutamate) ; poly(N-benzyloxycarbonyl-L-lysine)-b- polyoxyde d'éthylène ; polyoxyde d'éthylène -b- poly gamma acide L-glutamique, polyoxyde d'éthylène -b- poly (gamma Benzyl L-glutamate), les polydiméthylsiloxane -b-poly(L-acide glutamique); polydiméthylsiloxane-b-poly(Benzyl-Glutamate) ; poly(N-benzyloxycarbonyl-L-lysine)-b- poly(oxyde d'éthylène co oxyde de propylène) ; poly(oxyde d'éthylène co oxyde de propylène)-b- poly gamma acide L-glutamique, poly(oxyde d'éthylène co oxyde de propylène)-b- poly (gamma Benzyl L-glutamate) et leurs sels,
- des triblocs COIL-bloc-ROD-bloc-COIL ou ROD-b-COIL-b-ROD comme par exemple les poly(L-acide glutamique)-b-polydiméthylsiloxane-b- poly(L-acide glutamique) ; poly (gamma Benzyl L-glutamate)-b- poly(oxyde d'éthylène co oxyde de propylène)-b- poly (gamma Benzyl L-glutamate) ; poly(Benzyl-Glutamate )-b-polydiméthylsiloxane-b- poly(Benzyl-Glutamate) ; poly(L-valine)-b-poly(oxyde d'éthylène co oxyde de propylène)-b-poly(L-valine) et leurs sels.

9. Composition selon l'une quelconque des revendications précédentes, **caractérisée en ce qu'**elle contient 0,5 % en poids à 90 % en poids, en particulier de 0,7 % en poids à 85 % en poids, et plus particulièrement de 0,8 % en poids à 75 % en poids de copolymère(s) par rapport au poids total de la composition.

10. Composition selon l'une quelconque des revendications précédentes, **caractérisée en ce qu'**elle comprend au moins une phase aqueuse.

11. Composition selon l'une quelconque des revendications précédentes, **caractérisée en ce que** ladite composition comprend au moins une phase grasse.

12. Composition selon l'une quelconque des revendications 1 à 9 et 11, **caractérisée en ce qu'**elle est anhydre.

13. Composition selon la revendication 11 ou 12, **caractérisée en ce que** ladite phase grasse contient au moins un corps gras liquide à température ambiante et à pression atmosphérique et/ou au moins un corps gras solide à température ambiante et à pression atmosphérique.

14. Composition selon la revendication 13, **caractérisée en ce que** ledit corps gras liquide à température ambiante et à pression atmosphérique comprend au moins une huile volatile ou non volatile ou un de leurs mélanges.

15. Composition selon la revendication 13 ou 14, **caractérisée en ce que** ledit corps gras liquide à température ambiante et à pression atmosphérique représente de 0,01 à 90 % en poids, notamment de 0,1 à 85 % en poids par rapport au poids total de la phase grasse.

16. Composition selon l'une quelconque des revendications 13 à 15, **caractérisée en ce que** ledit corps gras solide à température ambiante et à pression atmosphérique est choisi parmi les cires, les corps gras pâteux, les gommes et leurs mélanges.

17. Composition selon l'une quelconque des revendications 11 à 16, **caractérisée en ce que** ladite phase grasse contient au moins un corps gras solide à raison de 0,01 à 50 %, notamment de 0,1 à 40 %, et en particulier de 0,2 à 30 %, en poids par rapport au poids total de la composition.

18. Composition selon l'une quelconque des revendications précédentes, **caractérisée en ce que** ladite composition comprend en outre une phase particulaire à raison de 0,01 à 40 %, notamment de 0,01 à 30 %, et en particulier de 0,05 à 20 %, en poids par rapport au poids total de ladite composition.

19. Composition selon la revendication 18, **caractérisée en ce que** ladite phase particulaire comprend au moins un pigment et/ou au moins une nacre et/ou au moins une charge complémentaire.

20. Composition selon l'une quelconque des revendications 1 à 11 et 13 à 19, **caractérisée en ce qu'**elle se présente sous forme d'une émulsion directe ou inverse.

21. Composition selon l'une quelconque des revendications précédentes, **caractérisée en ce qu'**elle se présente sous la forme de produits coulés en stick ou en coupelle.

22. Composition selon l'une quelconque des revendications précédentes, **caractérisée en ce qu'**elle se présente sous la forme d'un produit de maquillage et/ou de soin pour la peau et/ou les lèvres.

23. Composition selon l'une quelconque des revendications 1 à 20, **caractérisée en ce qu'**elle se présente sous la forme d'un produit de soin et/ou de maquillage des ongles.

24. Composition selon l'une quelconque des revendications 1 à 20, **caractérisée en ce qu'**elle se présente sous la forme d'un produit de soin et/ou de coiffage du cheveu.

25. Procédé de traitement cosmétique d'une matière kératinique comprenant au moins l'application sur ladite matière d'une composition selon l'une quelconque des revendications 1 à 24.

26. Utilisation d'un copolymère tel que défini selon l'une quelconque des revendications 1 à 8 à titre de tensioactifs.

27. Utilisation d'un copolymère tel que défini selon l'une quelconque des revendications 1 à 8 à titre d'agent rhéologique.

## Claims

1. A cosmetic composition containing, in a physiologically acceptable medium, at least one rod-coil type block copolymer comprising at least one "coil" polymeric block structure of variable conformation bonded to at least one "rod" block structure of restricted conformation, the composition being **characterized in that** said rod block structure is of polymeric nature and is constituted in full or in part of peptide motifs or the like with some or all of the free hydrogen atoms of said peptide motifs participating in non-covalent hydrogen bonds within the rod structure, said rod-block structure being or being derived from:
• poly(L-leucine),poly(L-valine), poly(phenylalanine),
• poly(L-lysine) and derivatives thereof, such as poly(N-benzyloxycarbonyl-L-lysine) and poly(N-trifluoroacetyl-L-lysine), and salts thereof such as hydrochlorides;
• poly(L-glutamic acid) and salts thereof such as the sodium salt, and derivatives thereof, such as poly γ-alkylesters in the range C₁-C₃₀ of L-glutamic acid such as poly(γ-methyl-L-glutamate) or poly γ-aryl esters in the range C₁-C₃₀ or poly γ-alkyl aryl esters in the range C₁-C₃₀ such as poly(γ-benzyl-L-glutamate),
• polyglutamine and derivatives thereof, such as poly(N-hydroxyethyl-L-glutamine and poly(N-hydroxypropyl-L-glutamine), and
• polypeptide copolymers of the above monomers of the poly(hydroxyethyl-L-glutamine and leucine), poly(hydroxyethyl-L-glutamine and valine), poly(γ-benzyl-L-glutamate and leucine), poly(γ-benzyl-L-glutamate and D,L-phenylalanine, poly(γ-benzyl-L-glutamate and cinnamylglutamate), poly(N-benzyloxycarbonyl-L-lysine and γ-benzyl-L-glutamate) type, and salts and derivatives thereof;
and said coil polymer being selected from:
· polyethers of the ethylene polyoxide type, propylene polyoxide type and copolymers thereof;
· homopolymers of siloxane, such as polydimethylsiloxane (PDMS) and polymethylphenylsiloxane, or polymethyllaurylsiloxane;
· copolymers of different types of the above polymers;
· copolymers of different types of the above polymers with other copolymers, such as for example polysiloxanes and polyoxide ethylene, and
· salts and derivatives thereof.

2. A composition according the preceding claim, **characterized in that** the number average molecular mass of the rod blocks lies in the range 200 g/mol to 1,000,000 g/mol, in particular 250 g/mol to 800,000 g/mol, and more particularly 250 g/mol to 500,000 g/mol.

3. A composition according to claim 1 or 2, **characterized in that** the rod blocks are present at at least 10%, in particular at least 15%, or at least 30%, and in particular at most 90%, notably at most 85%, even at most 80% by weight relative to the total weight of the copolymer.

4. A composition according to any one of claims 1 to 3, **characterized in that** the mean distance between the ends of a chain in the coil block, i.e. <R₀²>_{coil} satisfies the convention:
<R₀²>_{coil} = N L²
where L represents the length of a momomer and N represents the number of monomers constituting the block.

5. A composition according to any one of claims 1 to 4, **characterized in that** the number average molecular mass of the coil block lies in the range 300 g/mol to 1,000,000 g/mol, in particular 500 g/mol to 800,000 g/mol, and more particularly 500 g/mol to 500,000 g/mol.

6. A composition according to any one of claims 1 to 5, **characterized in that** its overall number average molecular mass lies in the range 700 g/mol to 1,000,000 g/mol, in particular 1,000 g/mol to 800,000 g/mol, and more particularly 2,000 g/mol to 500,000 g/mol.

7. A composition according to any one of claims 1 to 6, **characterized in that** said rod-coil block copolymer is not cross-linked.

8. A composition according to any one of claims 1 to 7, **characterized in that** said rod-coil copolymer is selected from the group comprising:
· rod-block-coil di-block (also abbreviated as rod -b- coil), such as for example ethylene polyoxide -b-poly(gamma benzyl L-glutamate); poly(N-benzyloxycarbonyl-L-lysine) -b- ethylene polyoxide; ethylene polyoxide -b-poly(gamma L-glutamic acid), polydimethylsiloxane -b-poly(L-glutamic acid), polydimethylsiloxane -b- poly(benzyl-glutamate), poly(N-benzyloxycarbonyl-L-lysine) -b- poly(ethylene oxide co propylene oxide), poly(ethylene oxide co propylene oxide) -b- poly gamma L-glutamic acid, poly(ethylene oxide co propylene oxide) -b- poly(gamma benzyl L-glutamate), and salts thereof;
· coil-block-rod-block-coil or rod -b- coil -b- rod tri-blocks such as for example poly(L-glutamic acid) -b-polydimethylsiloxane -b- poly(L-glutamic acid), poly(gamma benzyl L-glutamate) -b- poly(ethylene oxide co propylene oxide) -b- poly(gamma benzyl L-glutamate), poly(benzyl-glutamate) -b- polydimethylsiloxane -b-poly(benzyl-glutamate), poly(L-valine) -b- poly(ethylene oxide co propylene oxide) -b- poly(L-valine), and salts thereof.

9. A composition according to any preceding claim, **characterized in that** it contains 0.5% by weight to 90% by weight, in particular from 0.7% by weight to 85% by weight, and more particularly from 0.8% by weight to 75% by weight of copolymer(s) relative to the total weight of the composition.

10. A composition according to any preceding claim, **characterized in that** it includes at least one aqueous phase.

11. A composition according to any preceding claim, **characterized in that** said composition includes at least one fatty phase.

12. A composition according to any one of claims 1 to 9 and 11, **characterized in that** it is anhydrous.

13. A composition according to claim 11 or 12, **characterized in that** said fatty phase contains at least one fat that is liquid at ambient temperature and at atmospheric pressure, and/or at least one fat that is solid at ambient temperature and at atmospheric pressure.

14. A composition according to claim 13, **characterized in that** said fat that is liquid at ambient temperature and at atmospheric pressure comprises at least one volatile or non-volatile oil or a mixture thereof.

15. A composition according to claim 13 or claim 14, **characterized in that** said fat that is liquid at ambient temperature and at atmospheric pressure represents 0.01% to 90% by weight, and in particular 0.1% to 85% by weight relative to the total weight of the fatty phase.

16. A composition according to any one of claims 13 to 15, **characterized in that** said fat that is solid at ambient temperature and at atmospheric pressure is selected from waxes, pasty fats, gums, and mixtures thereof.

17. A composition according to any one of claims 11 to 16, **characterized in that** said fatty phase contains at least one solid fat constituting 0.01% to 50%, in particular 0.1% to 40%, and more particularly 0.2% to 30% by weight relative to the total weight of the composition.

18. A composition according to any preceding claim, **characterized in that** said composition further comprises a particulate phase constituting from 0.01% to 40%, in particular 0.01% to 30%, and more particularly 0.05% to 20% by weight relative to the total weight of said composition.

19. A composition according to claim 18, **characterized in that** said particulate phase comprises at least one additional pigment and/or nacre and/or filler.

20. A composition according to any one of claims 1 to 11 and 13 to 19, **characterized in that** it is in the form of an oil-in-water or a water-in-oil emulsion.

21. A composition according to any preceding claim, **characterized in that** it is in the form of a product that has been cast as a stick or a cake.

22. A composition according to any preceding claim, **characterized in that** it is in the form of a makeup and/or a care product for the skin and/or the lips.

23. A composition according to any one of claims 1 to 20, **characterized in that** it is in the form of a care product and/or a makeup for the nails.

24. A composition according to any one of claims 1 to 20, **characterized in that** it is in the form of a care product and/or a styling composition for the hair.

25. A method of cosmetically treating a keratinous material comprising at least applying a composition according to any one of claims 1 to 24 on said material.

26. The use of a copolymer as defined in any one of claims 1 to 8 as a surface active agent.

27. The use of a copolymer as defined in any one of claims 1 to 8 as a rheological agent.

## Patentansprüche

1. Kosmetische Zusammensetzung, enthaltend, in einem physiologisch verträglichen Medium, mindestens ein ROD-COIL-Blockcopolymer, umfassend mindestens eine polymere Blockstruktur mit variabler Konformation, die COIL genannt wird, die mit mindestens einer Blockstruktur mit eingeschränkter Konformation, die ROD genannt wird, verknüpft ist, **dadurch gekennzeichnet, dass** die ROD-Blockstruktur Polymercharakter aufweist und ganz oder zum Teil aus peptidischen Wiederholungseinheit(en) besteht, wobei die freien Wasserstoffatome der peptidischen Wiederholungseinheiten ganz oder zum Teil an nicht kovalenten Wasserstoffbrückenbindungen in der ROD-Struktur beteiligt sind,
wobei ROD-Blockstruktur folgende ist oder sich von folgendem ableitet:
- Poly-(L-leucin), Poly-(L-valin), Poly-(phenylalanin),
- Poly-(L-lysin), und seine Derivate, wie Poly-(N-benzyloxycarbonyl-L-lysin) und Poly-(N-trifluoracetyl-L-lysin) und ihre Salze, wie die Chlorhydrate
- Poly-(L-glutaminsäure), ihre Salze, wie das Natriumsalz, und ihre Derivate, wie die C₁-C₃₀-Poly-γ-alkylester der L-Glutaminsäure, wie Poly-(γ-methyl-L-glutamat), oder die C₁-C₃₀-Poly-γ-arylester oder die C₁-C₃₀-Poly-γ-alkylarylester, wie Poly-(γ-benzyl-L-glutamat),
- Polyglutamin und seine Derivate, wie Poly-(N-hydroxyethyl-L-glutamin) und Poly-(N-hydroxypropyl-L-glutamin), und
- polypeptidische Copolymere der vorhergehenden Monomere vom Typ Poly-(hydroxyethyl-L-glutamin/Leucin), Poly-(hydroxyethyl-L-glutamin/Valin), Poly-(γ-benzyl-L-glutamat/Leucin), Poly-(γ-benzyl-L-glutamat/D,L-Phenylalanin, Poly-(y-benzyl-L-glutamat/Cinnamylglutamat), Poly-(N-benzyloxycarbonyl-L-lysin/γ-Benzyl-L-glutamat), ihre Salze und Derivate;
und die COIL-Polymere ausgewählt sind aus:
- Polyethylenoxid-Polyethern, Polypropylenoxid-Polyethern und ihren Copolymeren,
- Siloxan-Homopolymeren, wie beispielsweise Polydimethylsiloxan (PDMS), und Polymethylphenylsiloxan oder Polymethyllaurylsiloxan,
- Copolymeren von verschiedenen Typen von vorgenannten Polymeren,
- Copolymeren von verschiedenen Typen von vorgenannten Polymeren mit anderen Copolymeren, wie beispielsweise Polysiloxan/Polyethylenoxid, und
- ihren Salzen und Derivaten.

2. Zusammensetzung nach dem vorhergehenden Anspruch, **dadurch gekennzeichnet, dass** die zahlenmäßige Masse der ROD-Blöcke von 200 g/mol bis 1.000.000 g/mol, insbesondere von 250 g/mol bis 800.000 g/mol und spezieller von 250 g/mol bis 500.000 g/mol variiert.

3. Zusammensetzung nach Anspruch 1 oder 2, **dadurch gekennzeichnet, dass** die ROD-Blöcke in einem Anteil von mindestens 10 Gew.-%, insbesondere von mindestens 15 Gew.-%, vor allem von mindestens 30 Gew.-% und insbesondere in einem Anteil von höchstens 90 Gew.-%, insbesondere von höchstens 85 Gew.%, sogar von höchstens 80 Gew.-% bezogen auf das Gesamtgewicht des Copolymers vorhanden sind.

4. Zusammensetzung nach einem der Ansprüche 1 bis 3, **dadurch gekennzeichnet, dass** der mittlere Abstand zwischen den Kettenenden in dem COIL-Block, nämlich <R₀²>_{COIL}, der Konvention gehorcht:
<R₀²>_{COIL} N L²,
wobei L die Länge von einem Monomer repräsentiert und N die Anzahl von den Block aufbauenden Monomeren repräsentiert.

5. Zusammensetzung nach einem der Ansprüche 1 bis 4, **dadurch gekennzeichnet, dass** die zahlenmäßige Masse der COIL-Blöcke von 300 g/mol bis 1.000.000 g/mol, insbesondere von 500 g/mol bis 800.000 g/mol und noch spezieller von 500 g/mol bis 500.000 variiert.

6. Zusammensetzung nach einem der Ansprüche 1 bis 5, **dadurch gekennzeichnet, dass** ihre zahlenmäßige Gesamtmasse von 700 g/mol bis 1.000.000 g/mol, insbesondere von 1000 g/mol bis 800.000 g/mol und noch spezieller von 2000 g/mol bis 500.000 g/mol variiert.

7. Zusammensetzung nach einem der Ansprüche 1 bis 6, **dadurch gekennzeichnet, dass** das ROD-COIL-Blockcopolymer nicht vernetzt ist.

8. Zusammensetzung nach einem der Ansprüche 1 bis 7, **dadurch gekennzeichnet, dass** das ROD-COIL-Blockcopolymer aus der Gruppe ausgewählt ist, die folgendes einschließt:
- ROD-Block-COIL-Zweierblöcke (auch als ROD-b-COIL abgekürzt), wie beispielsweise Polyethylenoxid-b-Poly-(gamma-benzyl-L-glutamat); Poly-(N-benzyloxycarbonyl-L-lysin)-b-Polyethylenoxid; Polyethylenoxid-b-Poly-(gamma-benzyl-L-glutaminsäure, Polyethylenoxid-b-Poly-(gamma-benzyl-L-glutamat); Polydimethylsiloxan-b-Poly-(L-glutaminsäure); Polydimethylsiloxan-b-Poly-(benzylglutamat); Poly-(N-benzyloxycarbonyl-L-lysin)-b-Poly-(ethylenoxid-propylencooxid); Poly-(ethylenoxid-propylencooxid)-b-Poly-gamma-L-glutaminsäure; Poly-(ethylenoxid-propylencooxid)-b-Poly-(gamma-benzyl-L-glutamat) und ihren Salzen,
- COIL-Block-ROD-Block-COIL-Dreierblöcken oder ROD-b-COIL-b-ROD, wie beispielsweise Poly-(L-glutaminsäure)-b-Polydimethylsiloxan-b-Poly-(glutaminsäure); Poly-(gamma-benzyl-L-glutamat)-b-Poly-(ethylenoxid-propylen-cooxid)-b-Poly-(gamma-Benzyl-L-glutamat); Poly-(benzylglutamat)-b-Polydimethylsiloxan-b-Poly-(benzylglutamat); Poly-(L-valin)-b-Poly-(ethylenoxid-propylencooxid)-b-Poly-(L-valin) und ihren Salzen.

9. Zusammensetzung nach einem der vorhergehenden Ansprüche, **dadurch gekennzeichnet, dass** sie 0,5 Gew.% bis 90 Gew.-%, insbesondere von 0,7 Gew.-% bis 85 Gew.-%, und noch spezieller von 0,8 Gew.-% bis 75 Gew.-% Copolymer(e) bezogen auf das Gesamtgewicht der Zusammensetzung enthält.

10. Zusammensetzung nach einem der vorhergehenden Ansprüche, **dadurch gekennzeichnet, dass** sie mindestens eine wässrige Phase umfasst.

11. Zusammensetzung nach einem der vorhergehenden Ansprüche, **dadurch gekennzeichnet, dass** die Zusammensetzung mindestens eine Fettphase umfasst.

12. Zusammensetzung nach einem der Ansprüche 1 bis 9 und 11, **dadurch gekennzeichnet, dass** sie wasserfrei ist.

13. Zusammensetzung nach Anspruch 11 oder 12, **dadurch gekennzeichnet, dass** die Fettphase mindestens einen bei Umgebungstemperatur und bei Atmosphärendruck flüssigen Fettkörper und/oder mindestens einen bei Umgebungstemperatur und bei Atmosphärendruck festen Fettkörper enthält.

14. Zusammensetzung nach Anspruch 13, **dadurch gekennzeichnet, dass** der bei Umgebungstemperatur und bei Atmosphärendruck flüssige Fettkörper mindestens ein flüchtiges oder nicht flüchtiges Öl oder eines von ihren Gemischen umfasst.

15. Zusammensetzung nach Anspruch 13 oder 14, **dadurch gekennzeichnet, dass** der bei Umgebungstemperatur und bei Atmosphärendruck flüssige Fettkörper 0,01 bis 90 Gew.-%, insbesondere 0,1 bis 85 Gew.-%, bezogen auf das Gesamtgewicht der Fettphase, repräsentiert.

16. Zusammensetzung nach einem der Ansprüche 13 bis 15, **dadurch gekennzeichnet, dass** der bei Umgebungstemperatur und bei Atmosphärendruck feste Fettkörper aus Wachsen, pastösen Fettkörper, Gummen und ihren Gemischen ausgewählt ist.

17. Zusammensetzung nach einem der Ansprüche 11 bis 16, **dadurch gekennzeichnet, dass** die Fettphase mindestens einen festen Fettkörper in einem Anteil von 0,01 bis 50 Gew.-%, insbesondere von 0,1 bis 40 Gew.-% und spezieller von 0,2 bis 30 Gew.-% bezogen auf das Gesamtgewicht der Zusammensetzung enthält.

18. Zusammensetzung nach einem der vorhergehenden Ansprüche, **dadurch gekennzeichnet, dass** die Zusammensetzung weiterhin eine teilchenförmige Phase in einem Anteil von 0,01 bis 40 Gew.-%, insbesondere von 0,01 bis 30 Gew.-% und insbesondere von 0,05 bis 20 Gew.-% bezogen auf das Gesamtgewicht der Zusammensetzung enthält.

19. Zusammensetzung nach Anspruch 18, **dadurch gekennzeichnet, dass** die teilchenförmige Phase mindestens ein Pigment und/oder mindestens einen Perlmuttlack und/oder mindestens einen zusätzlichen Füllstoff umfasst.

20. Zusammensetzung nach einem der Ansprüche 1 bis 11 und 13 bis 19, **dadurch gekennzeichnet, dass** sie in Form einer direkten oder inversen Emulsion vorliegt.

21. Zusammensetzung nach einem der vorhergehenden Ansprüche, **dadurch gekennzeichnet, dass** sie in Form von zu einem Stift oder zu einer Kuppelle gegossenen Produkten vorliegt.

22. Zusammensetzung nach einem der vorhergehenden Ansprüche, **dadurch gekennzeichnet, dass** sie in Form eines Schminkprodukts und/oder eines Pflegeprodukts für die Haut und/oder die Lippen vorhanden ist.

23. Zusammensetzung nach einem der Ansprüche 1 bis 20, **dadurch gekennzeichnet, dass** sie in Form eines Pflegeprodukts und/oder Übermalprodukts für die Nägel vorhanden ist.

24. Zusammensetzung nach einem der Ansprüche 1 bis 20, **dadurch gekennzeichnet, dass** sie in Form eines Pflegeprodukts und/oder eines Frisierprodukts für das Haar vorhanden ist.

25. Verfahren zur kosmetischen Behandlung einer keratinischen Substanz umfassend mindestens das Aufbringen einer Zusammensetzung nach einem der Ansprüche 1 bis 24 auf die Substanz.

26. Verwendung von einem Copolymer, wie nach einem der Ansprüche 1 bis 8 definiert, als grenzflächenaktives Mittel.

27. Verwendung von einem Copolymer, wie nach einem der Ansprüche 1 bis 8 definiert, als rheologisches Mittel.
